Europäisches Patentamt

(19) European Patent Office

Office européen des brevets

(11) Publication number: **0 084 169**

**B1**

(12) **EUROPEAN PATENT SPECIFICATION**

(45) Date of publication of patent specification: **04.09.85**

(51) Int. Cl.⁴: **A 61 K 9/50**

(21) Application number: **82112025.0**

(22) Date of filing: **27.12.82**

(54) **Method for the oral administration of substances in the form of liposomes, and relative pharmaceutical compositions.**

(30) Priority: **06.01.82 IT 1901282**

(43) Date of publication of application:
**27.07.83 Bulletin 83/30**

(45) Publication of the grant of the patent:
**04.09.85 Bulletin 85/36**

(84) Designated Contracting States:
**AT BE CH DE FR GB IT LI LU NL SE**

(56) References cited:
**AU-A- 53 107**

**CHEMICAL ABSTRACTS, vol. 90, no. 11, 12th March 1979, page 465, no. 85527h, Columbus Ohio (USA);**

(73) Proprietor: **AUSONIA FARMACEUTICI S.r.l.**
**Via Laurentina Km. 24730**
**I-00040 Pomezia (Rome) (IT)**

(72) Inventor: **Borgo, Eraldo**
**Via Tripoli 306**
**Ficarazzi di Catania Catania (IT)**

(74) Representative: **Bianchetti, Giuseppe**
**Studio Consulenza Brevettuale Via Senato 24**
**I-20121 Milan (IT)**

Courier Press, Leamington Spa, England.

## Description

It is known that liposomes are bodies having a microscopic size (diameter in the range of μm) forming in special conditions when substances having amphophilic character are placed in aqueous medium. Substances having amphophilic characters are those containing in the molecule a polar moiety and a non polar hydrophobic moiety, not soluble in water.

Typical amphophilic substances are phospholipids, or lecithines, and with these substances, as one of the main component, liposome preparations are prepared.

It is also known that many processes for the preparation of liposomes exist and that the exact structure of the liposomes depends on the preparation method. A typical process for the preparation of liposomes include evaporating a phospholipid solution, in admixture or not with other substances, for example cholesterol, in large surface vessel. When the solid has deposited a thin layer on the vessel's walls, an aqueous solution is added; by stirring an emulsion is formed wherein liposomes are present; by sonication the further formation of smaller liposomes, having more uniform characteristics, is obtained.

The liposomes so obtained are sphaeroidal bodies, with vesicular structure, mono or multilamellar, containing in their interior a certain amount of the aqueous solution in which they have been prepared: the aqueous solution in the interior of the vesicules is effectively separated from the rest of the solvent by the phospholipid layers and in such a way substances soluble in water can be sequestered in the liposomes themselves.

The preparations of liposomes containing in their interior pharmacologically active substances hold a great practical importance because entrapping in liposomes can modify absorption, the kinetics and the metabolism of the substances themselves when administered to animals or man. Particularly, the substances administered in liposomes by oral route, can be protected from enzymatic and non-enzymatic degradation, at gastro-enteric level, and can be directly absorbed through the intestinal wall, carried by liposomes themselves.

The practical use of preparations of liposomes is however hindered by numerous difficulties, some of which are advantageously overcome by the present invention.

Particularly, it is necessary to point out the limited stability in time of liposome preparation, the difficulty in producing large amounts of such preparations in a homogeneous way, and the scarcity of hydrosoluble material which can be normally entrapped in them.

It has now surprisingly found that particular solid or semisolid compositions of phospholipids containing hydrosoluble substances which are biologically or pharmacologically active, when administered as substantially anhydrous chewing tablets, form, during the chewing process, liposomes in large amounts, containing in their interior the biologically active substances. In this way the extemporaneous formation of liposomes is thus obtained together with englobation of the pharmacologically interesting substances.

Accordingly, this invention relates to pharmaceutical compositions for oral administration by means of liposomes of biologically active substances being in the form of substantially anhydrous chewing tablets containing hydrosoluble biologically active substances mixed with phospholips.

The substantially anhydrous chewing fomulations prepared according to the invention allow—because of the extemporaneous formation of liposomes containing the active principle—therapeutic results clearly better than those obtainable with other formulations administered by oral route. In the case of drugs subject to destruction in the digestive tract, and therefore administerable according to the previous art—only by parenteral route, the chewing formulations according to the invention have the big advantage of allowing the administration by oral route.

Of course, the formulations herein claimed can be administered both to man and to animals. The formulations according to the invention are obtained by mixing aqueous solutions containing the active principle with the phospholipids, and thereafter carrying out a more or less complete water evaporation, in such conditions to avoid the decomposition of heat-sensitive active principles; preferably the evaporation is carried out under reduced pressure, or in hot air or hot inert gas stream. According to a preferred embodiment, an aqueous solution of active principle is sprayed on granules of phospholipids (preferably of lecithin), and—contemporaneously or subsequently—the water is removed by passing a hot air stream through the mass of granules kept in mixing conditions. The granules so obtained are then worked up in tablets or lozenges optionally coated with inert substances, of the kind normally used in pharmaceutical technique.

The present invention is illustrated by the following example, which however must not be absolutely considered limitative of the invention itself, which must be considered extended to analogous embodiments according to the background art.

Example

Preparation of chewing tablets containing phospholipids and cytidin-diphosphocholine or other hydrosoluble and biologically active substances

1 kg of commercial soyabean lecithin in granular form, preferably of the kind used in human feeding, is used as additive. The granules have a solid or semi-solid consistancy. A concentrated (>10% in weight) aqueous solution of cytidin-diphosphocholine of the kind used in pharmaceutical preparations, is also prepared. The amount of the cytidin-diphosphocholine aqueous solution containing 200 g of the substance is sprayed on 1 kg of lecithin granules, while these are continuously mixed. During or after spraying, the water is evaporated in hot air stream going on with mixing so as to keep the granular aspect of the material. When the water is completely removed, the material is prepared in form of tablets weighing 0.5—1 g and the tablets are optionally coated with an inert substance, for instance mannitol. The ratio cytidin-diphosphocholine/lecithin can vary within large limits, but preferably it must not exceed the value of 1 to 3 by weight. Also the amount of water in which the cytidin-diphosphocholine is dissolved can vary but preferably the solution should be as concentrated as possible to make easy the further elimination of the water.

According to the above mentioned method formulations containing other hydrosoluble substances can be worked up, with results similar to those obtained for cytidin-diphosphocholine. For example formulations containing phospho-creatine, formulations containing δ-adenosyl-methionine, formulations containing sulphated polysaccharides and formulations of hydrosoluble vitamins and cobalamin have been prepared.

Demonstration of liposomes formation from the tablets during chewing

The demonstration of the formation of liposomial particles during chewing of the tablets prepared as previously described is obtained in the following way.

A tablet is subjected to chewing by volunteers; during chewing the material is not ingested, but retained in the buccal cavity; at the end of a normal chewing time the content is poured in a glass.

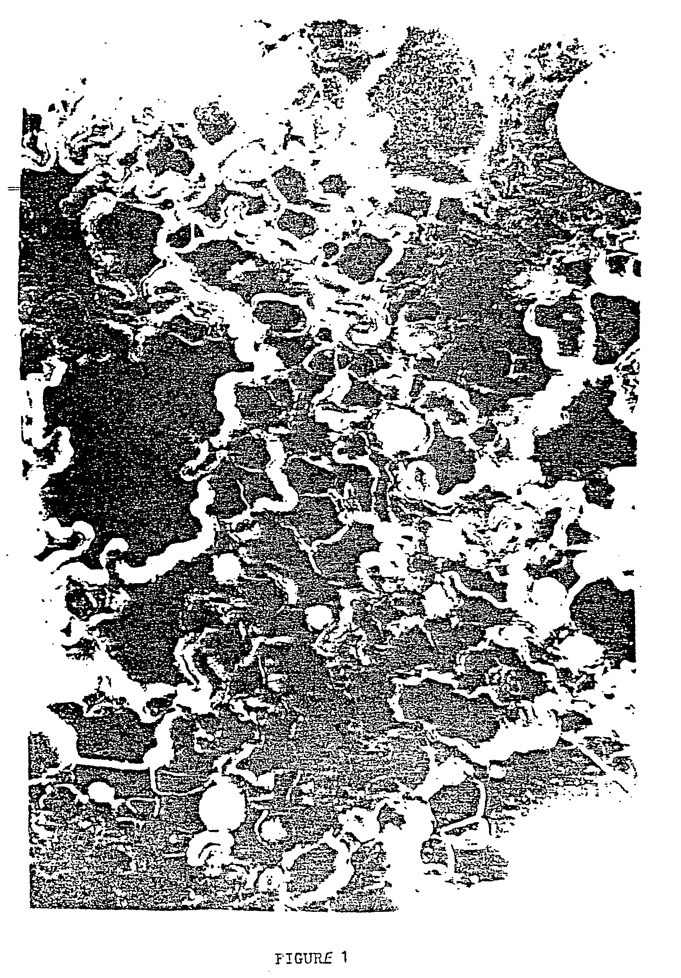

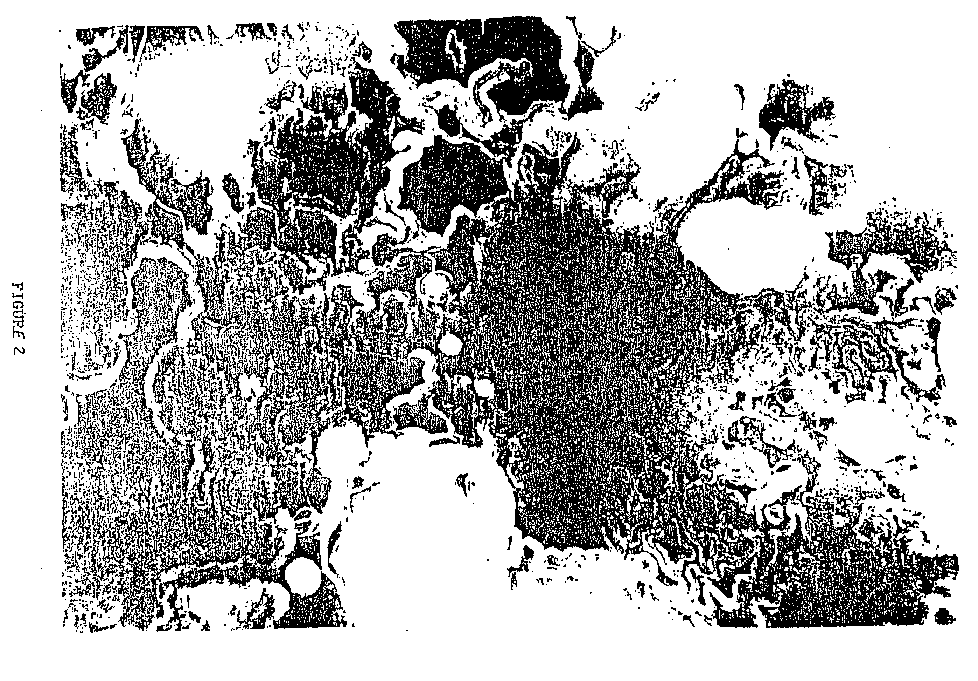

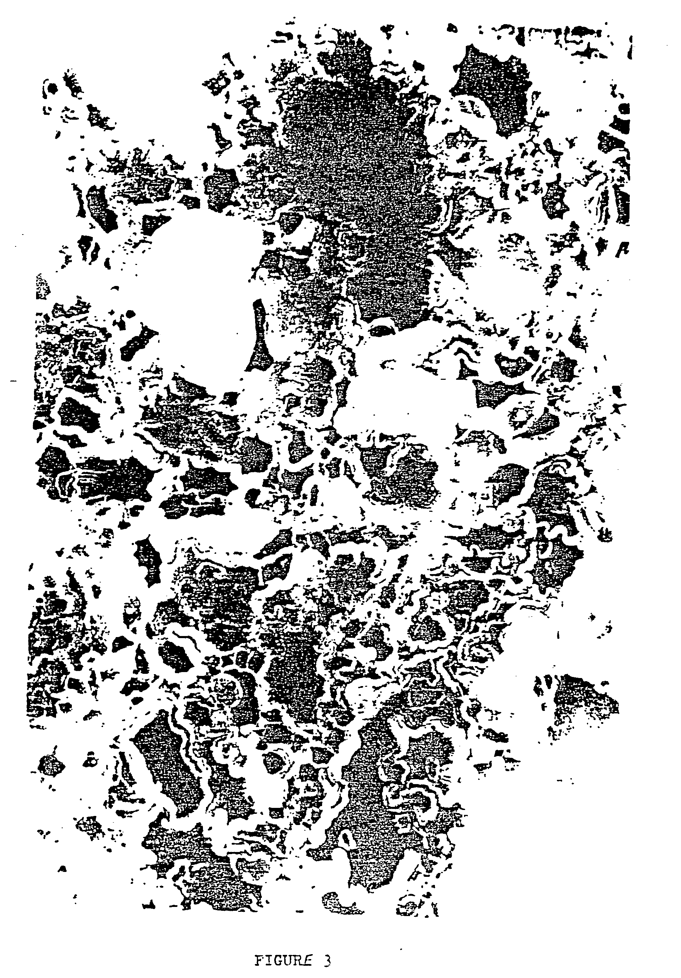

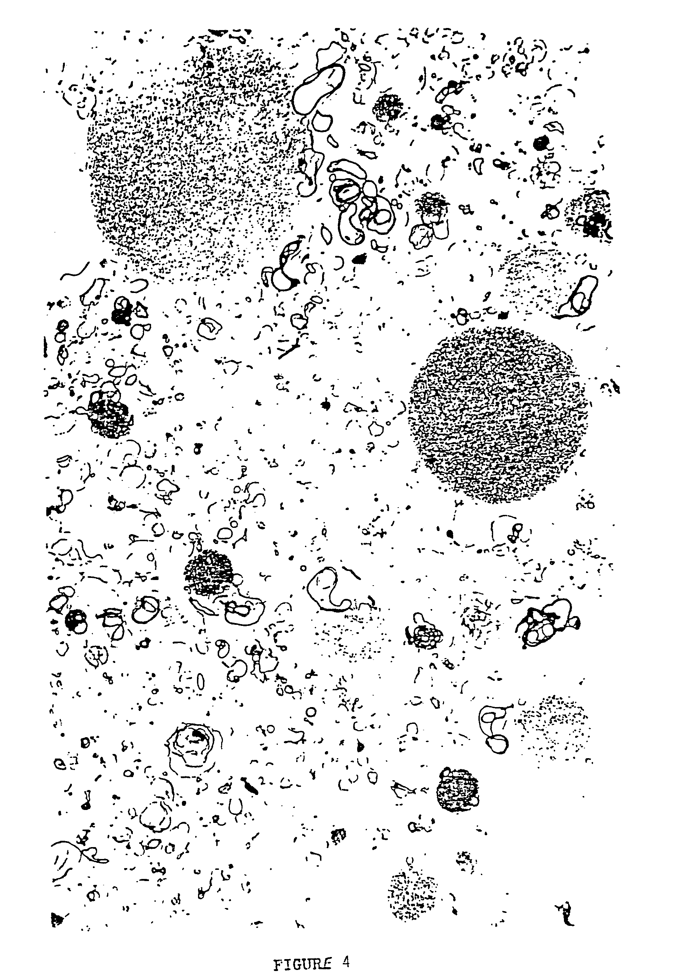

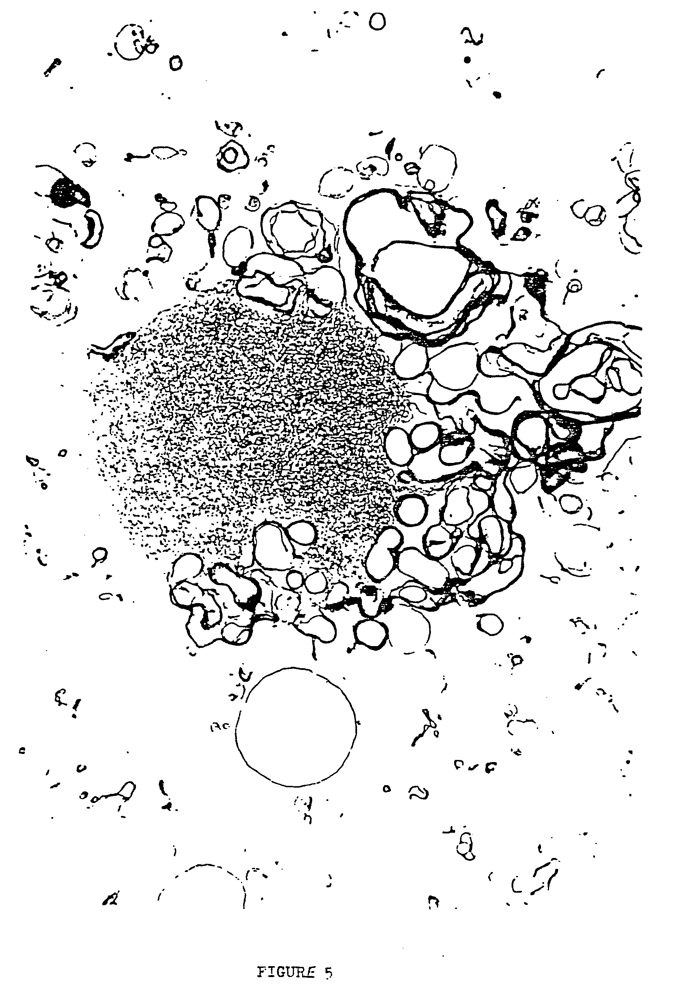

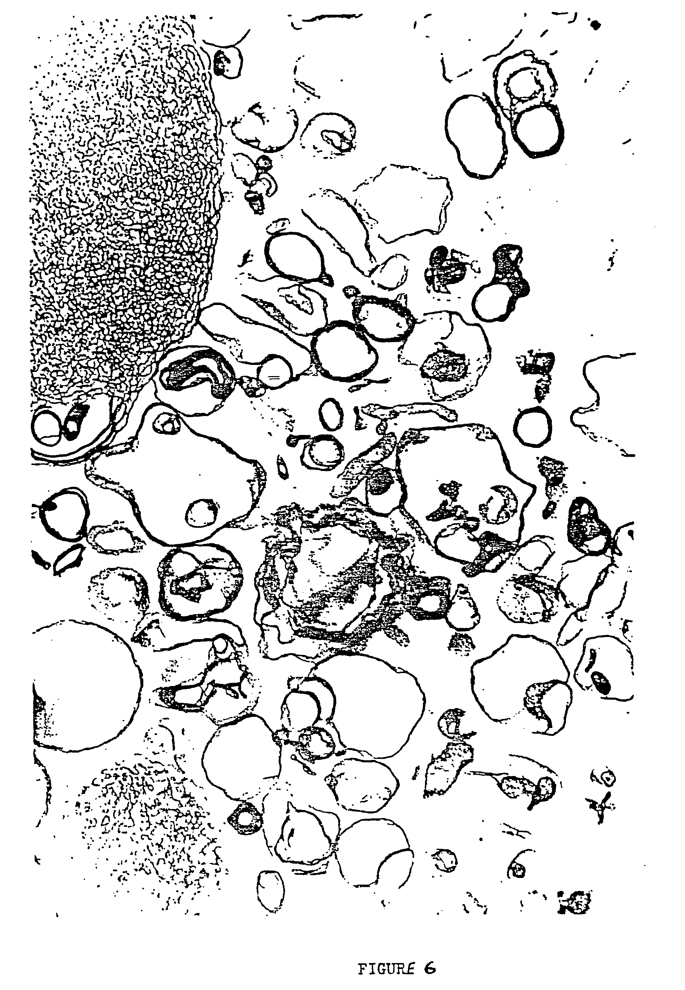

It is a very opalescent and viscous liquid in which only a few macroscopic granules are present. The observation of the microscopic particles suspended in the liquid is carried out by optical or electronic microscopy. Particularly illustrative are the pictures taken by electronic microscopy. They show the maximum presence of liposomial mono or multi-lamellar formations in form of vesicles having a diameter of some μm or less (see Figures 1—6).

By high-speed centrifugation the liposomial particles of the solution can be separated.

Measures of partition of cytidin-diphosphocholine between the aqueous phase and the vesicles show that a remarkable fraction of the cytidin-diphosphocholine initially contained in the tablets has been englobated in the liposomial particles.

**Claims for the Contracting States BE, CH, DE, FR, GB, IT, LI, LU, NL, SE**

1. Pharmaceutical compositions, suitable for the oral administration by means of liposomes of biologically active substances, characterized in that they are in the form of substantially anhydrous chewing tablets containing hydrosoluble biologically active substances mixed with phospholipids.

2. Pharmaceutical compositions according to claim 1, characterized in that they contain, as phospholipid, lecithin.

3. Pharmaceutical compositions according to claim 2, characterized in that the lecithin is in form of solid or semi-solid granules.

4. Pharmaceutical formulations according to claims 1—3, characterized in that the hydrosoluble biologically active substances comprise cytidin-diphosphocholine, phosphocreatine, δ-adenosyl-methionine, sulphated polysaccharides and/or hydrosoluble vitamins.

5. Pharmaceutical compositions according to claims 1—4, characterized in that they are tablets or lozenges, optionally coated with inert substances of the kind normally used in pharmaceutical technique.

6. Process for the preparation of pharmaceutical formulations according to claims 1—5, characterized in that the phospholipids are mixed with aqueous solutions of biologically active substances and that the mixture so obtained is subjected to dehydration.

7. Process according to claim 6, characterized in that lecithin is used as phospholipid.

8. Process according to claim 7, characterized in that lecithin is in form of solid or semi-solid granules.

**Claims for the Contracting State AT**

1. Process for the preparation of chewing pharmaceutical compositions in form of tablets allowing the administration by oral route of hydrosoluble biologically active compounds entrapped in liposomes such as cytidin-diphosphocholine, phosphocreatine, δ-adenoxyl-methionine, sulphated polysaccharides and/or hydrosoluble vitamins, characterized in that phospholipids are mixed with aqueous solutions of said biologically active substances and that the mixture so obtained is subjected to dehydration.

2. Process according to claim 1, characterized in that lecithin is used as phospholipid.

3. Process according to claim 2, characterized in that lecithin is in form of solid or- semi-solid granules.

**Patentansprüche für die Vertragsstaaten BE, CH, DE, FR, GB, IT, LI, LU, NL, SE**

1. Pharmazeutische Zusammensetzungen, geeignet für die orale Verabreichung durch Liposome biologisch aktiver Substanzen, dadurch gekennzeichnet, daß sie Form von im wesentlichen wasserfreien Kautabletten vorliegen, welche wasserlösliche biologisch aktive Substanzen gemischt mit Phospholipiden enthalten.

2. Pharmazeutische Zusammensetzungen nach Anspruch 1, dadurch gekennzeichnet, daß sie als Phospholipid Lecithin enthalten.

3. Pharmazeutische Zusammensetzungen nach Anspruch 2, dadurch gekennzeichnet, daß das Lecithin in Form fester oder halffester Körnchen vorliegt.

4. Pharmazeutische Formulierungen nach den Ansprüchen 1—3, dadurch gekennzeichnet, daß die wasserlöslichen biologisch aktiven Substanzen Cytidin-diphosphocholin, Phosphocreatin, δ-Adenosyl-methionin, sulfatierte Polysaccharide und/oder wasserlösliche Vitamine einschließen.

5. Pharmazeutische Zusammensetzungen nach den Ansprüchen 1 bis 4, dadurch gekennzeichnet, daß sie als Tabletten oder Pastillen vorliegen, gegebenenfalls überzogen mit inerten Substanzen der Art, wie sie normalerweise in der Pharmazeutik gebraucht werden.

6. Verfahren zur Herstellung von pharmazeutische Formulierungen nach den Ansprüchen 1 bis 5, dadurch gekennzeichnet, daß die Phospholipide mit wäßrigen Lösungen von biologisch aktiven Substanzen gemischt werden und die so erhaltene Mischung der Dehydratation unterworfen wird.

7. Verfahren nach Anspruch 6, dadurch gekennzeichnet, daß Lecithin als Phospholipid verwandt wird.

8. Verfahren nach Anspruch 7, dadurch gekennzeichnet, daß das Lecithin in Form fester oder halbfester Körnchen vorliegt.

**Patentansprüche für den Vertragsstaat AT**

1. Verfahren zur Herstellung von pharmazeutischen Zusammensetzungen zum Kauen in Form von Tabletten, welche die Verabreichung von wasserlöslichen biologisch aktiven Verbindungen, die in Liposome wie Cytidin-diphosphocholin, Phosphocreatin, δ-Adenosyl - methionin, sulfatierten Polysacchariden und/oder wasserlöslichen Vitaminen eingeschlossen sind, über den oralen Weg erlauben, dadurch gekennzeichnet, daß Phospholipide mit wäßrigen Lösungen dieser biologisch aktiven Verbindungen gemischt werden und die so erhaltene Mischung der Dehydratation unterworfen wird.

2. Verfahren nach Anspruch 1, dadurch gekennzeichnet, daß Lecithin als Phospholipid verwandt wird.

3. Verfahren nach Anspruch 2, dadurch gekennzeichnet, da Lecithin in Form von festen oder halbfesten Körnchen verwandt wird.

**Revendications pour les Etats Contractants BE, CH, DE, FR, GB, IT, LI, LU, NL, SE**

1. Compositions pharmaceutiques convenant à l'administration par voie orale, au moyen de liposomes, de substances biologiquement actives, caractérisées en ce qu'elles se présentent sous, la forme de tablettes à mastiquer sensiblement anhydres contenant des substances hydrosolubles biologiquement actives en mélange avec des phospholipides.

2. Compositions pharmaceutiques selon la revendication 1, caractérisées en ce qu'elles contiennent, à titre de phospholipide, de la lécithine.

3. Compositions pharmaceutiques selon la revendication 2, caractérisées en ce que la lécithine se présente sous la forme granulés solides ou semi-solides.

4. Compositions pharmaceutiques selon les revendications 1—3, caractérisées en ce que les substances hydrosolubles biologiquement actives comprennent la cytidine-diphosphocholine, la phosphocréatine, la δ-adénosyl-méthionine, les polysaccharides sulfatés et/ou des vitamines hydrosolubles.

5. Compositions pharmaceutiques selon la revendications 1 à 4, caractérisées en ce qu'elles sont constituées par des comprimés ou des pastilles éventuellement enrobés de substances inertes du type de celles habituellement utilisées dans la technique pharmaceutique.

6. Procédé de préparation des compositions pharmaceutiques selon les revendications 1 à 5, caractérisé en ce qu'il consiste à mélanger les phospholipides avec des solutions aqueuses de substances biologiquement actives et à soumettre à une déshydratation le mélange ainsi obtenu.

7. Procédé selon la revendication 6, caractérisé en ce que l'on utilise la lécithin à titre de phospholipide.

8. Procédé selon la revendication 7, caractérisé en ce que la lécithine se présente sous la forme de granulés solides ou semi-solides.

**Revendications pour l'Etat Contractant AT.**

1. Procédé de préparation de compositions pharmaceutiques à mastiquer se présentant sous la forme de comprimés permettant l'administration par voie orale de composés hydrosoluble biologiquement actifs emprisonnés dans des liposomes, tels que la citydine-diphosphocholine, la phosphocréatine, la δ-adénosyl-méthionine, des polysaccharides sulfatés et/ou des vitamines hydrosolubles, caractérisé en ce qu'il consiste à mélanger des phospholipides avec des solutions aqueuses desdites substances biologiquement actives et à soumettre à une déshydratation le mélange ainsi obtenu.

2. Procédé selon la revendication 1, caractérisé en ce qu'on utilise la lécithine à titre de phospholipide.

3. Procédé selon la revendication 2, caractérisé en ce que la lécithine se présente sous la forme de granulés solides ou semi-solides.

FIGURE 1

FIGURE 2

2

FIGURE 3

FIGURE 4

FIGURE 5

FIGURE 6